# EUROPÄISCHE PATENTSCHRIFT

(11) **EP 0 683 503 B1**
(45) Veröffentlichungstag und Bekanntmachung des Hinweises auf die Patenterteilung: **11.02.1998**
(21) Anmeldenummer: 95106541.6
(22) Anmeldetag: 29.04.1995
(51) Int. Cl.: H01J 49/02, G01N 33/00

(54) **Verfahren zur Auswertung der Signale eines Massenspektrometers und eines Sensors**
Method for processing signals from a mass spectrometer and a detector
Procédé d'exploitation des signaux d'un spectromètre de masse et d'un capteur

(30) Priorität: 20.05.1994 CH 1571/94; 03.04.1995 CH 926/95
(43) Veröffentlichungstag der Anmeldung: 22.11.1995
(73) Patentinhaber: ELPATRONIC AG, CH-6303 Zug (CH)
(72) Erfinder: Alvarez, Fernando, CH-5415 Rieden (CH); Dr. ROSATZIN Martin, 8424 Embrach (CH)

(56) Entgegenhaltungen:
- EP-A- 0 306 307
- D. L. MASSART ET AL. 'CHEMOMETRICS: A TEXTBOOK' 1988 , ELSEVIER , AMSTERDAM * Seite 159, Absatz 1.4 - Seite 162; Abbildung 3 * * Seite 319 - Seite 321; Abbildungen 3,4 *
- INTERNATIONAL JOURNAL OF MASS SPECTROMETRY AND ION PROCESSES., Bd. 89, 1989 AMSTERDAM NL, Seiten 111-124, S. KÖRNIG ET AL. 'TANDEM MASS SPECTROMETRY RESOLUTION ENHANCEMENT BY MULTIVARIATE ANALYSIS'
- CHEMOMERTICS AND INTELLIGENT LABORATORY SYSTEMS, Bd. 22, Nr. 1, Januar 1994 AMSTERDAM, Seiten 63-76, W. WERTHER ET AL. 'CLASSIFICATION OF MASS SPECTRA , A COMPARISON OF YES/NO CLASSIFICATION METHODS FOR THE RECOGNITION OF SIMPLE STRUCTURAL PROPERTIES'

## Beschreibung

Die Erfindung betrifft ein Verfahren gemäss Oberbegriff des Anspruchs 1. Ferner betrifft die Erfindung eine Anwendung des Verfahrens nach Anspruch 3.

Es ist bekannt, Signale aus einer Mehrzahl von Sensoren derart auszuwerten, dass jedem Sensorsignal einer oder mehrere Schwellenwerte zugeordnet werden. Dies setzt eine Auswerteschaltung oder ein Auswerteprogramm mit entsprechend vielen Kanälen voraus. Andererseits ist es bekannt, Massenspektren und Kernresonanzspektren durch Musterverarbeitungsverfahren oder durch Expertensysteme (z.B. das Expertensystem DENDRAL für Kernresonanzspektren) zu verarbeiten. Dabei werden eine Vielzahl von Informationen und deren Verknüpfung im Sinne einer Mustererkennung und eines Mustervergleichs erfasst.

Aus International Journal of Mass Spectrometry and Ion Processes, Bd. 89, 1989, S. 111-124, S. Körnig et al., "Tandem mass spectrometry resolution enhancement by multivariate analysis" ist es bekannt, mit zwei Massenspektrometern zu arbeiten, deren Spektrensignale gemeinsam dargestellt werden. Aus D.L. Massart et al., "Chemometrics: a Textbook" 1988, Amsterdam, Seiten 159-162 ist ein "multivavelength detector" bekannt.

Keines dieser Dokumente betrifft hingegen die Auswertung von Massenspektren und zusätzlichen Signalen, die durch andere Sensoren als Massenspektrometer gewonnen worden sind. Es hat sich indes gezeigt, dass für gewisse Anwendungen die Verwendung eines Massenspektrometers einerseits und mindestens eines Sensors andererseits (z.B. eines Sensors für einen speziellen Stoff, für den das Massenspektrometer relativ geringe Empfindlichkeit aufweist) vorteilhaft ist. Indes bedingt die zweifache Auswertung sowohl des Spektrums als auch des Sensorsignals einen zusätzlichen Aufwand.

Der Erfindung liegt die Aufgabe zugrunde, die Signale eines Massenspektrometers und von Sensoren auf einfache und rasche Weise auswerten zu können. Dies wird mit den kennzeichnenden Merkmalen des Anspruchs 1 erreicht.

Die Sensorsignale werden in dieses Spektrum aufgenommen, wie wenn es sich bei diesen Signalen um weitere Spektralwerte handeln würde und das so ergänzte Spektrum wird als Ganzes ausgewertet. Dadurch entfällt der zusätzliche Auswertungsaufwand.

Eine bevorzugte Anwendungsart des Verfahrens liegt bei der Prüfung von Behältern, insbesondere von Mehrweg-Flaschen. Dort ist es bereits bekannt, Massenspektrometer einzusetzen, welche eine Gasprobe aus dem Behälter analysieren, und das ermittelte Spektrum mittels Mustererkennung auszuwerten. Es wäre nun wünschenswert, für bestimmte Stoffe, z.B. für polyzyklische aromatische Kohlenwasserstoffe (PAK), welche auf die genannte Weise relativ schlecht nachweisbar sind, handelsübliche separate Sensoren einzusetzen. Dies ist an sich ohne weiteres möglich, bedingt aber eine separate Auswertung des Sensorsignals. Soll das Sensorsignal mit der Angabe des PAK-Wertes zudem in Relation mit der Massenspektrogrammauswertung gesetzt werden, so ergibt sich ein grosser Aufwand, insbesondere da bei der Prüfung von Mehrweg-Flaschen im industriellen Umfeld nur eine sehr kurze Zeit zur Verfügung steht (z.B. 100 msec). Die Anwendung des erfindungsgemässen Verfahrens ergibt dabei eine sehr elegante und einfache Lösung. Das Signal des PAK-Sensors wird als ein weiterer Massebalken in das Spektrogramm aufgenommen und dieses wird - wie bekannt - mittels Musterverarbeitung ausgewertet. Die separate zusätzliche Sensorauswertung kann so vermieden werden und die Musterverarbeitung ermöglicht zudem auf einfache Weise das in Beziehung setzen des PAK-Massebalkens zu den anderen Massebalken des Spektrums.

Im folgenden werden Ausführungsbeispiele anhand der Zeichnungen näher erläutert. Dabei zeigt
Figur 1 schematisch die Anordnung zur Auswertung der Signale mehrerer Sensoren;
Figur 2 ebenfalls schematisch die Darstellung der Sensorsignale;
Figur 3 die Darstellung von Sensorsignalen bei der Prüfung einer Mehrweg-Flasche; und
Figur 4 eine Darstellung gemäss Figur 3 mit zusätzlicher Anfügung eines Farbspektrums.

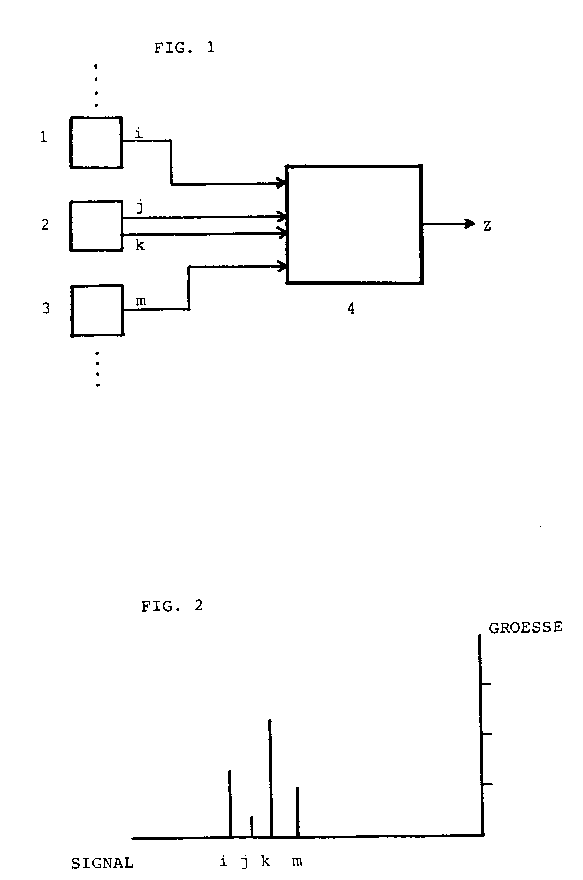

Figur 1 zeigt schematisch das Prinzip einer Auswertung der Signale mehrerer Signalgeber. Dabei sind als Beispiel drei Signalgeber 1,2,3 gezeigt, welche die Signale i; j; k und m abgeben, und von welchen der Signalgeber 1 ein Massenspektrum ist; die übrigen Signalgeber können beliebige Sensoren oder Analysengeräte sein, welche als Signale beliebige Messwerte liefern. In der Regel liegen die Signale als elektrische Signale vor. In einer Auswerteinrichtung 4 (in der Regel ein Rechner) werden die Signale aufbereitet, in eine einheitliche Darstellung gebracht und dann z.B. mittels Mustererkennung verarbeitet. Je nach Verarbeitungsresultat wird ein Ausgangssignal Z ausgegeben.

Gemäss der Erfindung werden die verschiedenen Sensorsignale in das Spektrum des Massenspektrometers eingefügt. Zum Beispiel werden die Signale j, k und l zu einem Muster angeordnet und zu dem Spektrum i(welches in der Zeichnung vereinfachend nur mit einem Balken dargestellt ist) hinzugefügt. Das resultierende Spektrum muss für die Auswertung durch den Rechner natürlich nicht in für den Menschen sichtbarer Form vorliegen, sondern es genügt, wenn die Darstellung maschinenlesbar ist.

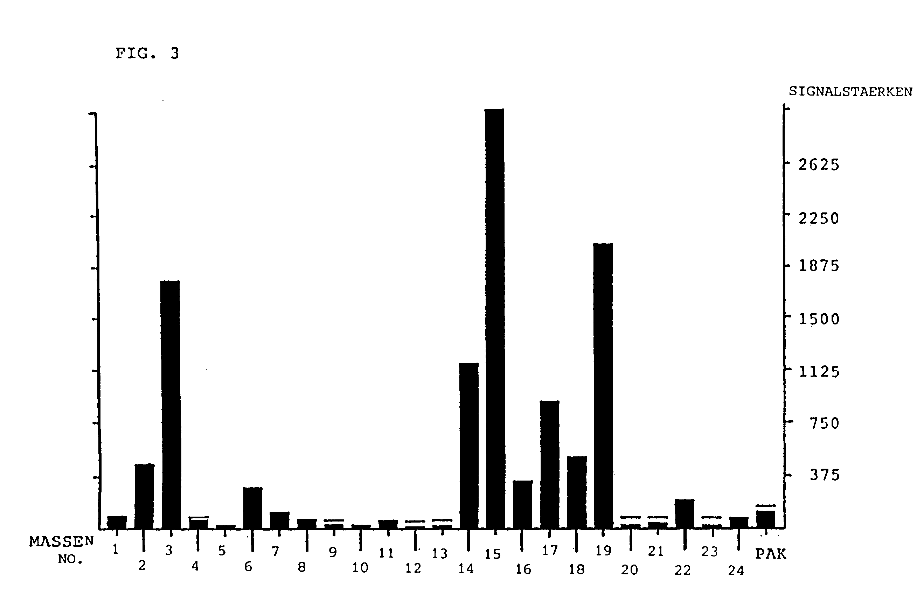

Figur 3 zeigt ein Ausgangssignal eines Massenspektrometers, welches z.B. bei der Prüfung von aus Behältern entnommenen Gasproben eingesetzt wird, wobei die Behälter insbesondere rücklaufende Mehrweg-Flaschen sind, die auf störende Verunreinigungen geprüft werden. Je nachdem ob eine massenspektroskopisch ermittelte Verunreinigung vorliegt oder nicht, wird die Flasche aus dem Mehrweg-Umlauf ausgeschieden oder nicht. Dies ist bekannt und es ist auch bekannt, das jeweils ermittelte Spektrogramm mittels Musteranalyse auszuwerten, um den Ausscheideentscheid zu fällen. Das Spektrogramm ist dabei in Figur 3 nur zum Zwecke der Erläuterung optisch sichtbar dargestellt. Zum Zwecke der maschinellen Analyse braucht das Spektrum nur in maschinenlesbarer Form vorzuliegen, wobei aber das Muster als solches in der maschinenlesbaren Darstellung enthalten ist, man kann daher auch für die maschinenlesbare Darstellung von einer "graphischen" Darstellung sprechen. In dem gezeigten Beispiel ist nun das Signal eines weiteren Sensors, der kein Massenspektrometer ist und nur ein normales elektrisches Signal, z.B. im Bereich von 0 bis 1 Volt, abgibt, in die Spektrodarstellung eingefügt. Dazu wird von der Auswerteinrichtung 4 das Signal des Sensors nach vorgegebenen Regeln an die Skalierung des Spektrogrammes angepasst und an einer vorbestimmten Stelle, im gezeigten Beispiel am Ende des Spektrums, als Balken PAK eingesetzt. Das resultierende, in der Figur 3 optisch dargestellte Spektrum wird dann der Musteranalyse auf bekannte Weise unterworfen, wodurch das Sensorausgangssignal auf einfache Weise direkt mitanalysiert wird. Auf die Analyse eines Spektrums braucht hier nicht näher eingegangen zu werden, da entsprechende Musteranalyseprogramme bekannt sind. Das in Figur 3 gezeigte Sensorausgangssignal ist bei der Flaschenprüfung vorzugsweise das Ausgangssignal eines handelsüblichen Sensors für polyzyklische aromatische Kohlenwasserstoffe (PAK). Natürlich können auf die selbe Weise noch weitere Sensorausgangssignale verarbeitet werden. Auch diese werden skaliert dem Spektrum beigefügt und mit diesem analysiert. So kann z.B. auch ein spezieller Sensor für Ammoniak vorgesehen sein. Solche Sensoren (z.B. nach dem Chemoluminiszenzverfahren arbeitend) sind bekannt. Auch dieses Sensorausgangssignal wird in das Spektrum eingefügt.

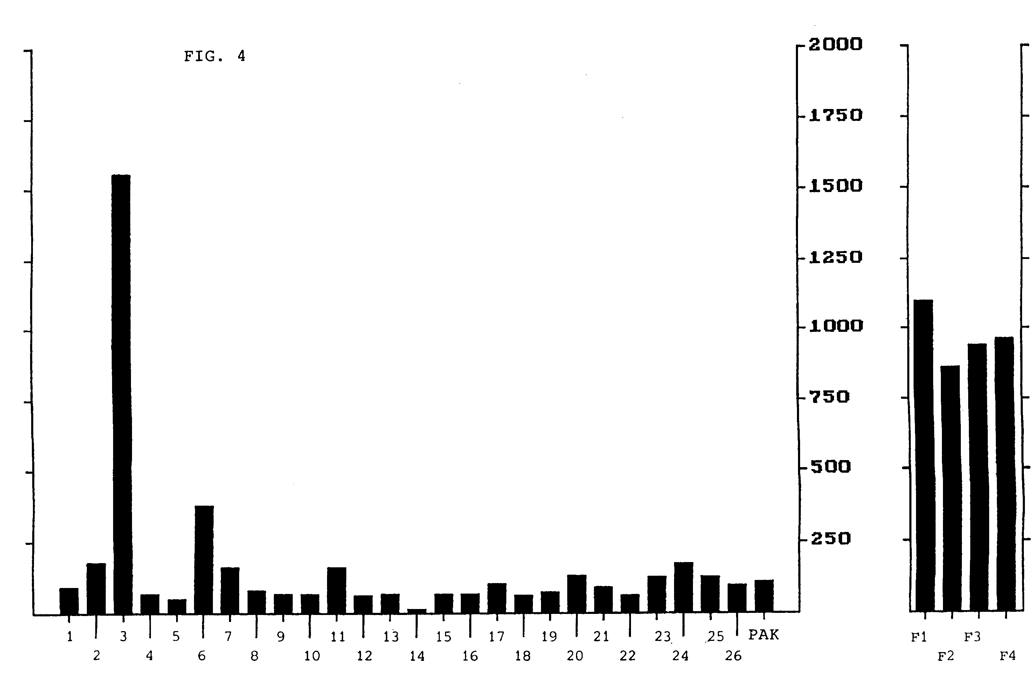

Figur 4 zeigt das Spektrum gemäss Figur 3, an welches vier Spektralwerte eines Farbsensors angefügt sind (die Farben F1-F4). Mittels des Farbsensors wird die Farbe der Restflüssigkeit untersucht (z.B. auf die Farben Rot, Grün, Blau hin). Auch dies lässt Schlüsse auf die Art der Restflüssigkeit zu und kann in Verbindung mit dem Massenspektrum ausgewertet werden. Die Auswertung erfolgt dabei mit dem im wesentlichen selben Algorithmus wie beim Massenspektrogramm.

## Patentansprüche

1. Verfahren zur Auswertung von Signalen mindestens zweier Signalgeber, dadurch gekennzeichnet, dass der erste Signalgeber ein ein Spektrum erzeugendes Massenspektrometer ist, dass der mindestens eine weitere Signalgeber, der kein Massenspektrometer ist, ein auf einen spezifischen Stoff oder auf die Eigenschaften eines Stoffes ansprechender Sensor ist, und dass das Signal des Sensors als Spektralwert bzw. -werte in das Spektrum eingefügt wird und das so ergänzte Spektrum als Ganzes ausgewertet wird.

2. Verfahren nach Anspruch 1, dadurch gekennzeichnet, dass das ergänzte Spektrum aus dem Massenspektrogramm einer Gasprobe und dem Signal eines die Gasprobe auf das Vorhandensein polyzyklischer aromatischer Kohlenwasserstoffe prüfenden Sensors und/oder eines auf Ammoniak ansprechenden Sensors gebildet wird, wobei das Sensorsignal als Massebalken in das Massenspektrogramm eingefügt wird.

3. Verfahren nach Anspruch 1 oder 2, dadurch gekennzeichnet, dass dem Spektrum das Signal mindestens eines Farbsensors angefügt wird.

4. Anwendung des Verfahrens nach einem der Ansprüche 1 bis 3 bei der Prüfung von Behältern, insbesondere Mehrweg-PET-Flaschen, auf Verunreinigungen, wobei die Signalgeber Analyseeinrichtungen für aus den Behältern entnommene Gasproben bzw. für in den Behältern befindliche Restflüssigkeit bilden.

5. Anwendung nach Anspruch 4, dadurch gekennzeichnet, dass eine Analyseeinrichtung in Form eines Massenspektrometers und eine Analyseeinrichtung in Form eines Sensors für polyzyklische aromatische Kohlenwasserstoffe und/oder eines Sensors für Ammoniak vorgesehen ist, welche Analyseeinrichtungen eine Gasprobe aus dem Behälter analysieren, und dass das oder die Sensorsignale in das Massenspektrogramm der Gasprobe als Massebalken eingefügt wird bzw. werden, und dass das so ergänzte Massenspektrogramm als Ganzes mittels eines Mustererkennungsverfahrens ausgewertet wird.

6. Anwendung nach Anspruch 4 oder 5, dadurch gekennzeichnet, dass eine Analyseeinrichtung eine Farbanalyse der Restflüssigkeit im Behälter durchführt.

## Claims

1. Method for interpreting signals from at least two signal transmitters, characterized in that the first signal transmitter is a mass spectrometer producing a spectrum, in that the at least one additional signal transmitter, which is not a mass spectrometer, is a sensor responding to a specific substance or to the properties of a substance, and in that the signal from the sensor is inserted in the spectrum as a spectral value or values and the spectrum thus produced is interpreted as a whole.

2. Method according to Claim 1, characterized in that an augmented spectrum is formed from the mass spectrogram of a gas sample and the signal of a sensor testing the gas sample for the presence of polycyclic aromatic hydrocarbons and/or the signal of a sensor responding to ammonia, the sensor signal being inserted into the mass spectrogram as a mass bar.

3. Method according to Claim 1 or Claim 2, characterized in that the signal from at least one colour sensor is added to the spectrum.

4. Application of the method according to any one of Claims 1 to 3 to the testing of containers, in particular returnable PET bottles, for contamination, in which the signal transmitters are analysers of gas samples taken from the containers and/or of residual liquid present in the containers.

5. Application according to Claim 4, characterized in that analysers in the form of a mass spectrometer and a sensor for polycyclic aromatic hydrocarbons and/or a sensor for ammonia are provided to analyse a gas sample from the container, and in that the sensor signal or signals is or are inserted in the mass spectrogram of the gas sample as a mass bar and in that the mass spectrogram thus produced is interpreted as a whole by a pattern recognition process.

6. Application according to Claim 4 or Claim 5, characterized in that an analyser performs a colorimetric analysis of the residual liquid in the container.

## Revendications

1. Procédé d'exploitation de signaux provenant d'au moins deux générateurs de signaux, caractérisé en ce que le premier générateur de signaux est un spectromètre de masse générant un spectre, en ce que ledit au moins un autre générateur de signaux, qui n'est pas un spectromètre de masse, est un capteur réagissant à une substance particulière ou aux propriétés d'une substance, et en ce que le signal émis par le capteur est introduit en tant que valeur ou valeurs spectrale(s) dans le spectre et en ce que le spectre ainsi complété est exploité sous la forme d'un tout.

2. Procédé selon la revendication 1, caractérisé en ce que le spectre complété est constitué à partir du spectrogramme de masse d'un échantillon de gaz et du signal provenant d'un capteur analysant l'échantillon de gaz à la recherche d'hydrocarbures aromatiques polycycliques et/ou d'un capteur réagissant à la présence d'ammoniac, le signal du capteur étant introduit dans le spectrogramme de masse en tant que raie du spectre de masse.

3. Procédé selon la revendication 1 ou 2, caractérisé en ce qu'il est adjoint au spectre le signal d'au moins un capteur de couleur.

4. Utilisation du procédé selon l'une des revendications 1 à 3 pour le contrôle de récipients, notamment de bouteilles de PET à plusieurs voies, à la recherche d'impuretés, les générateurs de signaux constituant des dispositifs d'analyse pour des échantillons de gaz prélevés dans les récipients ou pour le liquide résiduel présent dans les récipients.

5. Utilisation selon la revendication 4, caractérisée en ce qu'un dispositif d'analyse est prévu sous la forme d'un spectromètre de masse et un dispositif d'analyse sous la forme d'un capteur d'hydrocarbures aromatiques polycycliques et/ou d'un capteur d'ammoniac, dispositifs d'analyse qui analysent un échantillon de gaz provenant du récipient, et en ce que le signal ou les signaux provenant du capteur est ou sont introduit(s) dans le spectrogramme de masse de l'échantillon de gaz en tant que raie de spectre de masse, et en ce que le spectrogramme de masse ainsi complété est exploité sous la forme d'un tout au moyen d'un procédé de reconnaissance d'échantillons.

6. Utilisation selon la revendication 4 ou 5, caractérisée en que qu'un dispositif d'analyse effectue une analyse chromatique du liquide résiduel présent dans le récipient.
